# EUROPEAN PATENT SPECIFICATION

(11) **EP 3 060 674 B1**
(45) Date of publication and mention of the grant of the patent: **19.05.2021**
(21) Application number: 14796342.5
(22) Date of filing: 21.10.2014
(51) Int. Cl.: C12P 19/14, C12Q 1/48, C12P 7/48, C12P 7/56, C12P 13/08, C12P 13/14, G01N 33/48

(54) **ENHANCED FERMENTATION PROCESS USING A TRANSGLYCOSIDASE**
INTENSIVIERTER GÄRPROZESS MIT EINER TRANSGLYCOSIDASE
PROCESSUS DE FERMENTATION AMÉLIORÉ DANS LEQUEL ON UTILISE UNE TRANSGLYCOSIDASE

(30) Priority: 24.10.2013 WO PCT/CN2013/085866
(43) Date of publication of application: 31.08.2016
(73) Proprietor: Danisco US Inc., Palo Alto, California 94304 (US)
(72) Inventor: DUAN, Gang, Palo Alto, California 94304 (US); SHETTY, Jayarama K., Palo Alto, California 94304 (US); WANG, Xin, Palo Alto, California 94304 (US); XU, Hongxian, Palo Alto, California 94304 (US); ZHANG, Xiaoping, Palo Alto, California 94304 (US); ZHOU, Peng, Palo Alto, California 94304 (US)
(74) Representative: Mewburn Ellis LLP
(86) International application number: PCT/US2014/061529
(87) International publication number: WO 2015/061289

(56) References cited:
- WO-A1-2011/100161
- WO-A2-2009/134964
- WO-A2-2010/010463
- US-A1- 2009 142 812
- GUI-GUANG CHEN ET AL: "A high-throughput method for screening ofmutants with high transglycosylation activity by detecting non-fermentable reducing sugar", WORLD JOURNAL OF MICROBIOLOGY AND BIOTECHNOLOGY, KLUWER ACADEMIC PUBLISHERS, DO, vol. 27, no. 6, 4 November 2010 (2010-11-04), pages 1519-1523, XP019899327, ISSN: 1573-0972, DOI: 10.1007/S11274-010-0595-0
- ELISE CHAMPION ET AL: "A pH-Based High-Throughput Screening of Sucrose-Utilizing Transglucosidases for the Development of Enzymatic Glucosylation Tools", CHEMCATCHEM, vol. 2, no. 8, 22 July 2010 (2010-07-22), pages 969-975, XP055162145, ISSN: 1867-3880, DOI: 10.1002/cctc.201000111

## Description

### Field of the Invention

The present invention relates to methods and uses of a transglycosidase enzyme preparation for the reduction of foam and/or viscosity in the fermentation broth to improve fermentation processes.

### Description of the Related Art

Industrial biotechnology represents the third wave in biotechnology and a growing number of companies are now focusing on specialty chemicals as an entry point to build the bio-based economy. Glutamic acid, lysine, lactic acid are top targets for further research and development within industrial biotechnology. The objective of a bio-refinery is to develop as more product and value streams as possible from biomass. Background summaries of the current technologies of bio-refinery fermentation can be found in Eggeling, L. and M. Bott (2010). Handbook of Corynebacterium glutamicum, CRC press; Zahoor, A., S. N. Lindner, et al. (2012). "Metabolic engineering of Corynebacterium glutamicum aimed at alternative carbon sources and new products." Computational and Structural Biotechnology Journal 3(4); Ikeda, M. and S. Takeno (2013). "Amino Acid Production by Corynebacterium glutamicum." 23: 107-147.

Current challenges and objectives include maximizing product yield from raw materials. While much research has been carried out in this area, including strain modification, various raw materials utilization, and process optimization, yields remain far from theoretical maximums. Another current problem is managing the foaming level of aerobic fermentation. Under certain conditions, the existence of the foam phase in bioreactors may be regarded as a positive factor enhancing mass transfer and growth of the given culture. On the other hand, intensive foam formation frequently reduces the actual volume of the liquid. Furthermore, foam ejection and loss of the fermentation product may occur, which negatively impacts manufacturing plant capacity. Also, contamination occurs as a consequence of overflowing foam, so antifoam is often required to control the foam, which can be quite costly and negatively impact the downstream processing used to recover the end-products. In aerobic fermentation, often the maximum amount of fermentation broth per reactor is governed by foaming, thus filling degree (determined as mass per reactor volume) varies between 70 and 85% in industrial practices. Foaming is influenced by the density of the fermentation broth, surface composition of the production strain, media composition, process conditions, viscosity of the fermentation medium, and so on. Though there are some studies focusing on foam formation (see e.g. Eggeling, L., K. Krumbach, et al. (2001). "L-glutamate efflux with Corynebacterium glutamicum: why is penicillin treatment or Tween addition doing the same?" Journal of molecular microbiology and biotechnology 3(1): 67-68; Eggeling, L. and H. Sahm (2001). "The cell wall barrier of Corynebacterium glutamicum and amino acid efflux." Journal of bioscience and bioengineering 92(3): 201-213; Eggeling, L. and M. Bott (2010). Handbook of Corynebacterium glutamicum, CRC press; Yang, Y., F. Shi, et al. (2012). "Purification and structure analysis of mycolic acids in Corynebacterium glutamicum." The Journal of Microbiology 50(2): 235-240), there no reports on foaming reduction by using additional enzymes. Another area of current research is directed to improving downstream process efficiency of bio-refinery fermentations. Recovery processes of amino acids and organic acids from fermentation broth may include conventional chromatographic method, concentrated crystallization method, direct drying method, calcium salt precipitation, and so on. Centrifugation, filtration, ultra-filtration, crystallization, spray-drying, etc are also often used to recover fermentation

WO 2009/134964 A1 discloses methods of utilizing at least one transglucosidase enzyme to increase the amount of fermentable sugars in molasses fermentation processes; i.e. a method for increasing the fermentation yield of molasses in a fermentation medium which are converted to obtain end products, such as an alcohol (e.g., ethanol), an organic acid (e.g., succinic acid, lactic acid), sugar alcohols (e.g., glycerol), ascorbic acid intermediates (e.g., gluconate, DKG, KLG), and amino acids (e.g., lysine). Among suitable transglucosidases (D-glucosyltransferases, E.C. 2.4.1.25), the TG L-500 (commercially available from Genecor) or D45356 (GID:2645159; Aspergillus niger aglA gene for alpha-glucosidase) are mentioned.

US2009142812 A discloses the preparation of a high molecular weight, reduced viscosity starch paste adding one or more starch hydrolyzing enzymes; adding a second starch-hydrolyzing enzyme (a - amylase, beta amylase, glucoamylase, pullulanase and/or isoamylase) or adding a transglucosidase enzyme to the composition, namely A. niger from Genencor.

### Summary of the Invention

A method of making a fermentation product comprising; fermenting a feedstock with a fermenting microorganism in a fermentation broth to make a fermentation product, wherein the fermenting comprises a transglycosidase, that reduces foam and/or viscosity in the fermentation broth and wherein the fermentation product has a higher yield than a control fermentation lacking the transglycosidase. In some embodiments, the method further comprises recovering the fermentation product. In some embodiments, the fermentation product is lysine, lactic acid, or glutamic acid. In some embodiments, the fermenting organism is *Brevibacterium flavum, Corynebacterium glutamicum 10178, Corynebacterium glutamicum 10238, or Lactobacillus rhamnosus.* The transglycosidase comprises SEQ ID NO:1, or a sequence at least 80% identical to it. In other embodiments, the transglycosidase comrprises SEQ ID NO:1, or a sequence at least, 85%, 90%, 95%, 98%, 99% identical to it. The transglycosidase is produced by expression in a Trichoderma. In some embodiments, the feedstock comprises starch liquifact, granular starch, pure glucose, pure sucrose, or sugar syrup. In some embodiments, the Transglucosidase enzyme preparation (TrTG) is present at 1-6 Kg/10³Kg (Kg/MT), 2-5 Kg/10³Kg (Kg/MT), or 2-4 Kg/10³Kg (Kg/MT) dry solids of fermentation feedstock.

In some embodiments, the present teachings provide a method of making lysine comprising; fermenting a feedstock with *Brevibacterium flavum* in a fermentation broth to make lysine, wherein the fermenting comprises a TrTG, wherein the lysine has a higher yield compared to a control fermentation lacking the TrTG, and wherein the fermentation broth has reduced viscosity compared to a control fermentation lacking the TrTG.

In some embodiments, the present teachings provide a method of making lysine comprising; fermenting a feedstock with *Brevibacterium flavum* in a fermentation broth to make lysine, wherein the fermenting comprises an A. Niger TG, wherein the lysine has a higher yield compared to a control fermentation lacking the A. Niger TG.

In some embodiments, the present teachings provide a method of making lysine comprising; fermenting a feedstock with *Brevibacterium flavum* in a fermentation broth to make lysine, wherein the fermenting comprises a Transglucosidase, wherein the lysine has a higher yield compared to a control fermentation lacking the Transglucosidase. In some embodiments, the feedstock comprises pure glucose or sugar syrup.

In some embodiments, the present teachings provide a method of making lysine comprising; fermenting a feedstock with *Brevibacterium flavum* in a fermentation broth to make lysine, wherein the fermenting comprises a betaglucanase, xylanase and cellulase enzyme complex, wherein the lysine has a higher yield compared to a control fermentation lacking the betaglucanase, xylanase and cellulase enzyme complex.

In some embodiments, the present teachings provide a method of making glutamic acid comprising; fermenting a feedstock with *Corynebacterium glutamicum SP 10238* in a fermentation broth to make glutamic acid, wherein the fermenting comprises a TrTG, wherein the fermentation broth has reduced viscosity compared to a control fermentation lacking the TrTG, and wherein the fermentation broth has reduced foam compared to a control fermentation lacking the TrTG.

In some embodiments, the present teachings provide a method of making glutamic acid comprising; fermenting a feedstock with *Corynebacterium glutamicum SP 10238* in a fermentation broth to make glutamic acid, wherein the fermenting comprises an exoglucanase, a endoglucanase, a β-glucosidase, and a xylanase, wherein the fermentation broth has reduced viscosity compared to a control fermentation lacking the exoglucanase, the endoglucanase, the β-glucosidase, and the xylanase, and wherein the fermentation broth has reduced viscosity compared to a control fermentation lacking the exoglucanase, the endoglucanase, the β-glucosidase, and the xylanase.

In some embodiments, the present teachings provide a method of making lactic acid comprising; fermenting a feedstock with *Lactobacillus rhamnosus* in a fermentation broth to make lactic acid, wherein the fermenting comprises a TrTG, wherein the lactic acid has a higher yield compared to a control fermentation lacking the TrTG, and wherein the fermentation broth has reduced viscosity compared to a control fermentation lacking the TrTG. In some embodiments, the feedstock comprises pure glucose or sugar syrup.

In some embodiments, the present teachings provide a method of making lactic acid comprising; fermenting a feedstock with *Lactobacillus rhamnosus* in a fermentation broth to make lactic acid, wherein the fermenting comprises a betaglucanase, xylanase and cellulase enzyme complex, wherein the lactic acid has a higher yield compared to a control fermentation lacking the betaglucanase, xylanase and cellulase enzyme complex, and wherein the fermentation broth has reduced viscosity compared to a control fermentation lacking the betaglucanase, xylanase and cellulase enzyme complex.

More generally, the present invention involves adding transglucosidases enzymes during fine-chemicals fermentation. Increasing product yield, decreasing antifoam consumption, improving downstream efficiency. Without being bound by theory, the present inventors have found out that during bio-refinery fermentation, with the help of transglucosidase enzymes, beneficial effects may be found, including but not limited to, strain stimulation, accelerated fermentation rate, increased fermentation product yield, reduction in final residual sugars, decreased consumption of antifoam, decreased fermentation broth viscosity, and/or increased precipitation/settling speed.

### Brief Description of the Figures

Figure 1 depicts the effect of Transglucosidase on the settling of insolubles of fermentation broth using glucose syrup as substrate.
Figure 2 depicts the effect of TrTGL2000 on settling of insolubles of fermentation broth using pure glucose as substrate.
Figure 3 depicts the effect of TrGA on settling of insolubles of fermentation broth using glucose syrup as substrate.

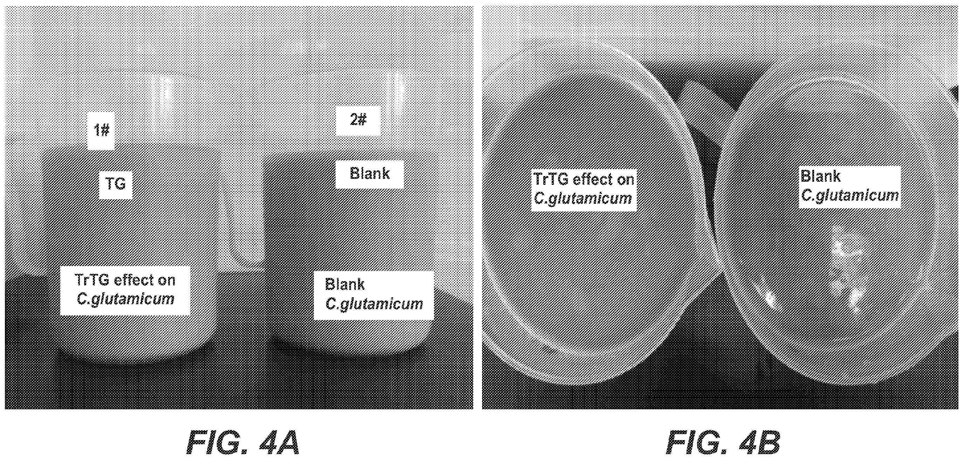

Figures 4A-B depict the effect of TrTG L2000 on settling of insolubles of fermentation broth using pure glucose as substrate, strain *Corynebacterium glutamicum 10178.*

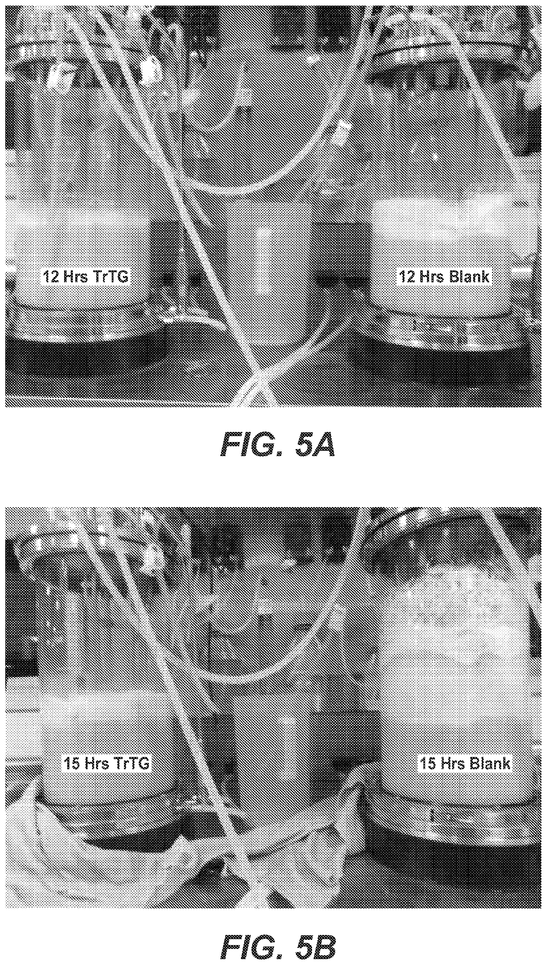

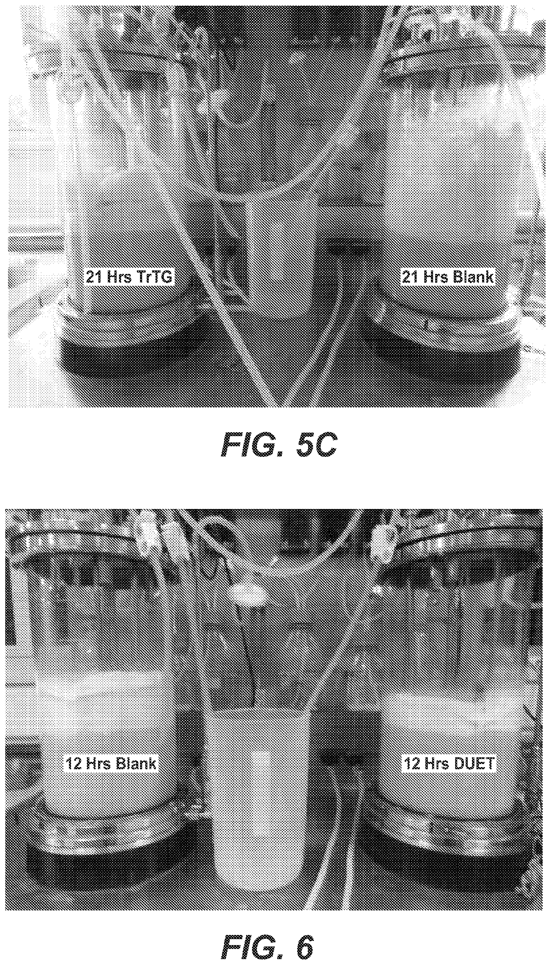

Figures 5A-C depict the effect of TrTG L2000 on foaming broth using pure glucose as substrate, strain *Corynebacterium glutamicum SP 10238.*
Figure 6 depicts the effect of ACCELLERASE DUET on foaming broth using pure glucose as substrate, strain *Corynebacterium glutamicum SP 10238.*

### Definitions

As used herein, the term "transglycosidase" refers to any enzyme or enzyme-containing preparation capable of transferring a monosaccharide moiety, as defined below, from one molecule to another. The term 'transglucosidase' is used when the monosaccharide moiety is a glucose moiety. In one embodiment, the transglycosidase enzyme is a transglucosidase enzyme. In one embodiment, the transglycosidase is classified in enzyme classification (E.C.) 3.2.1.24. In one embodiment, the transglycosidase enzyme is classified in Glycoside Hydrolase Family 31 of the Carbohydrate-Active Enzymes (CAZy) database. This database is described at http://www.cazy.org/ and in Coutinho, P. M. & Henrissat, B. (1999). This classification system is based on structural and sequence features rather than substrate specificity: as there is a direct relationship between sequence and folding similarities; such a classification: (i) reflects the structural features of these enzymes better than their sole substrate specificity, (ii) helps to reveal the evolutionary relationships between these enzymes, and (iii) provides a convenient tool to derive mechanistic information. In this classification, glycoside hydrolases (EC 3.2.1.-) are a widespread group of enzymes which hydrolyse the glycosidic bond between two or more carbohydrates or between a carbohydrate and a non-carbohydrate moiety. Further description of the classification of glycoside hydrolases (glycosidases and transglydosidases can be found in Henrissat B (1991), Henrissat B, Bairoch A (1993), Henrissat B, Bairoch A (1996), Davies G, Henrissat B (1995) and Henrissat B, Davies G J (1997)). In one embodiment, the transglycosidase enzyme is obtainable or is obtained from a living organism. Suitable transglycosidase enzymes are of bacterial or fungal origin. Preferred are transglycosidase enzymes of fungal origin. In one embodiment, the transglycosidase enzyme is of fungal origin or has at least 80%, at least 85%, at least 90%, at least 95%, at least 96%, at least 97%, at least 98% or at least 99%, sequence identity with the mature transglucosidase enzyme of fungal origin that is SEQ ID NO: 1 (herein referred to as a "TrTG", since the transglucosidase was made by expression in Trichoderma, as described generally in WO 09/114380) .

SEQ ID NO: 1
Asqsllsttapsqpqftipasadvgaqlianiddpqaadaqsvcpgykaskvqhnsrgftaslqlagrpcnvygtdvesltlsveyqds
drlniqilpthvdstnaswyflsenlvprpkaslnasvsqsdlfvswsnepsfnfkvirkatgdalfstegtvlvyenqfiefvtalpeeyn
lyglgehitqfrlqrnanltiypsddgtpidqnlygqhpfyldtryykgdrqngsyipvksseadasqdyislshgvflrnshgleillrsq
kliwrtlgggidltfysgpapadvtrqyltstvglpamqqyntlgfhqcrwgynnwsdladvvanfekfeipleyiwtdidymhgyr
nfdndqhrfsysegdeflsklhesgryyvpivdaalyipnpenasdayatydrgaaddvflknpdgslyigavwpgytvfpdwhhp
kavdfwanelviwskkvafdgvwydmsevssfcvgscgtgnltlnpahpsfllpgepgdiiydypeafnitnateaasasagassqa
aatatttstsvsylrttptpgvrnvehppyvinhdqeghdlsvhavspnathvdgveeydvhglyghqglnatyqgllevwshkrrpfi
igrstfagsgkwaghwggdnyskwwsmyysisqalsfslfdipmfgadtcgfngnsdeelcnrwmqlsaffpfyrnhnelstipqe
pyrwasvieatksamriryailpyfytlfdlahttgstvmralswefpndptlaavetqfmvgpaimvvpvleplvntvkgvfpgvgh
gevwydwytqaavdakpgvnttisaplghipvyvrggnilpmqepalttrearqtpwallaalgsngtasgqlylddgesiypnatlh
vdftasrsslrssaqgrwkernplanvtvlgvnkepsavtlngqavfpgsvtynstsqvlfvgglqnltkggawaenwvlew

A nucleic acid sequence encoding this protein is found in SEQ ID NO: 2.

SEQ ID NO: 2

tccaccactgccccttcgcagccgcagtttaccattcctgcttccgcagatgtcggtgcgcagctgattgccaacatcgatgatcctcaggct
gccgacgcgcagtcggtttgtccgggctacaaggcttcaaaagtgcagcacaattcacgtggattcactgccagtcttcagctcgcgggca
ggccatgtaacgtatacggcacagatgttgagtccttgacactgtctgtggagtaccaggattcggatcgactgaatattcagattctcccca
ctcatgttgactccacaaacgcttcttggtactttctttcggaaaacctggtccccagacccaaggcttccctcaatgcatctgtatcccagagc
gaccttttttgtgtcatggtcaaatgagccgtcgttcaatttcaaggtgatccgaaaggctacaggcgacgcgcttttcagtacagaaggcact
gtgctcgtatatgagaatcagttcatcgaatttgtgaccgcgctccctgaagaatataacttgtatggccttggggagcatatcacgcaattcc
gcctccagagaaatgctaatctgaccatatatccttcggatgatggaacacctattgaccagtgagtactgatatcccgcccgtatcttctggtt
ctactcttgaaacttactcgtcctagaaacctctacggccaacatcccttctatctggatacaagatattacaaaggagataggcagaatgggt
cttatattcccgtcaaaagcagcgaggctgatgcctcgcaagattatatctccctctctcatggcgtgtttctgaggaactctcatggacttgag
atactcctccggtctcaaaaattgatctggcggaccctaggtggaggaatcgatctcaccttctactcaggccccgccccggccgatgttac
caggcaatatcttaccagcactgtgggattaccggccatgcagcaatacaacactcttggattccaccaatgtcgttggggctacaacaact
ggtcggatctggcggacgttgttgcgaactttgagaagtttgagatcccgttggaatatatctggtgcgtattgtactggtttatggtatctcaaa
acagtctaacaggcacttaggaccgatattgactacatgcacggatatcgcaactttgacaacgatcaacatcgcttttcctacagtgagggc
gatgaatttctcagcaagctacatgagagtggacgctactatgtacccattgttgatgcggcgctctacattcctaatcccgaaaatgcctctg
atgcgtaagtgtctagtgacaaattatattactgcctgtatgctaattagcgatacagatacgctacgtatgacagaggagctgcggacgacg
tcttcctcaagaatcccgatggtagcctctatattggagccgtttggccaggatatacagtcttccccgattggcatcatcccaaggcagttga
cttctgggctaacgagcttgttatctggtcgaagaaagtggcgttcgatggtgtgtggtacgacatgtctgaagtttcatccttctgtgtcggga
gctgtggcacaggtaacctgactctgaacccggcacacccatcgtttcttctccccggtgagcctggtgatatcatatatgattacccagagg
ctttcaatatcaccaacgctacagaggcggcgtcagcttcggcgggagcttccagtcaggctgcagcaaccgcgaccaccacgtcgactt
cggtatcatatctgcggacaacgcccacgcctggtgtccgcaatgttgagcacccaccctatgtgatcaaccatgaccaagaaggccatga
tctcagtgtccatgcggtgtcgccgaatgcaacgcatgttgatggtgttgaggagtatgatgtgcacggtctctacggacatcaaggattgaa
cgctacctaccaaggtctgcttgaggtctggtctcataagcggcggccatttattattggccgctcaaccttcgctggctctggcaaatgggc
aggccactggggcggcgacaactattccaaatggtggtccatgtactactccatctcgcaagccctctccttctcacttttcggcattccgatg
tttggtgcggacacctgtgggtttaacggaaactccgatgaggagctctgcaaccgatggatgcaactgtccgcattcttcccattctaccga
aaccacaatgagctctccacaatcccacaggagccttatcggtgggcttctgttattgaagcaaccaagtccgccatgagaattcggtacgc
catcctaccttacttttatacgttgtttgacctggcccacaccacgggctccactgtaatgcgcgcactttcctgggaattccctaatgacccaa
cattggctgcggttgagactcaattcatggttgggccggccatcatggtggtcccggtattggagcctctggtcaatacggtcaagggcgta
ttcccaggagttggacatggcgaagtgtggtacgattggtacacccaggctgcagttgatgcgaagcccggggtcaacacgaccatttcg
gcaccattgggccacatcccagtttatgtacgaggtggaaacatcttgccgatgcaagagccggcattgaccactcgtgaagcccggcaa
accccgtgggctttgctagctgcactaggaagcaatggaaccgcgtcggggcagctctatctcgatgatggagagagcatctaccccaat
gccaccctccatgtggacttcacggcatcgcggtcaagcctgcgctcgtcggctcaaggaagatggaaagagaggaacccgcttgctaat

gtgacggtgctcggagtgaacaaggagccctctgcggtgaccctgaatggacaggccgtatttcccgggtctgtcacgtacaattctacgt
cccaggttctctttgttggggggctgcaaaacttgacgaagggcggcgcatgggcggaaaactgggtattggaatgg

In some embodiments, the transglycosidase enzyme originates from an *Aspergillus* species, especially an Aspergillus species selected from the group consisting of *Aspergillus niger* (herein referred to as an "A. niger TG"), *Aspergillus awamori, Aspergillus terreus, Aspergillus oryzae, Aspergillus nidulans, Aspergullus fumigatus and Aspergillus clavatus* or has at least 50%, preferably at least 55%, such as at least 60%, for example at least 65%, at least 70%, at least 75%, at least 80%, at least 85%, at least 90%, at least 95%, at least 96%, at least 97%, at least 98% or at least 99%, sequence identity with a transglucosidase enzyme originating from an *Aspergillus* species. In one embodiment, the transglycosidase enzyme is *Aspergillus niger* a sequence having at least 50%, preferably at least 55%, such as at least 60%, for example at least 65%, at least 70%, at least 75%, at least 80%, at least 85%, at least 90%, at least 95%, at least 96%, at least 97%, at least 98% or at least 99%, sequence identity therewith. The transglycosidase enzyme may be post-translationally modified, for example by cleavage of a signal sequence or by glycosylation.

As used herein the term "foam reduction" refers to the reduction of at least 1 centimeter of foam after 12 hours relative to a blank foam height condition as assessed according to the general conditions described in Example 6. In some embodiments, the foam reduction comprises at least 1cm, 2cm, 3cm, 4cm, or 5cm relative to a control fermentation lacking the transglycosidase.

As used herein the term "viscosity reduction" refers to a decrease in viscosity (mpas) as measured after 12 hours relative to a control group as assessed according to the general conditions described in Example 8. In some embodiments, the viscosity reduction comprises at least 1%, 5%, 10%, 20%, 25%, 30%, 40%, 50%, or 60% reduction relative to a control fermentation lacking the transglycosidase.

As used herein, the term "pure glucose" refers to at least 95% glucose.

As used herein, the term "pure sucrose" refers to at least 95% sucrose.

As used herein, the term "feedstock" refers to any carbon source that can be utilized by a fermenting microorganism to make a fermentation product. Illustrative feedstocks include pure glucose, pure sucrose, granular starch, liquefied starch, and any of a variety of cellulosic materials.

As used herein, "fermenting microorganism" refers to any microorganism, including bacterial and fungal organisms, suitable for use in a desired fermentation process to produce a fermentation product. The fermenting organism can be hexose and/or pentose fermenting organisms, or a combination thereof. Both hexose and pentose fermenting organisms are well known in the art. Suitable fermenting microorganisms are able to ferment, *i.e.,* convert, sugars, such as glucose, xylose, xylulose, arabinose, maltose, mannose, galactose, and/or oligosaccharides, directly or indirectly into the desired fermentation product. Examples of bacterial and fungal fermenting organisms producing ethanol are described by Lin et al., 2006, Appl. Microbiol. Biotechnol. 69: 627-642.

As used herein, "fermentation product" refers to any substance derived from the fermentation. The fermentation product can be, without limitation, an alcohol (*e.g*.,_arabinitol, n-butanol, isobutanol, ethanol, glycerol, methanol, ethylene glycol, 1,3-propanediol [propylene glycol], butanediol, glycerin, sorbitol, and xylitol); an alkane (*e.g.,* pentane, hexane, heptane, octane, nonane, decane, undecane, and dodecane), a cycloalkane (*e.g.,* cyclopentane, cyclohexane, cycloheptane, and cyclooctane), an alkene (*e.g.* pentene, hexene, heptene, and octene); an amino acid (*e.g*., aspartic acid, glutamic acid, glycine, lysine, serine, tryptophan, and threonine); a gas (*e.g.*, methane, hydrogen (H2), carbon dioxide (CO₂), and carbon monoxide (CO)); isoprene; a ketone (*e.g*., acetone); an organic acid (*e.g.,* acetic acid, acetonic acid, adipic acid, ascorbic acid, citric acid, 2,5-diketo-Dgluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid,oxaloacetic acid, propionic acid, succinic acid, and xylonic acid); 1-3 propane diol, and polyketide. The fermentation product can also be protein as a high value product.

In addition to those explicitly exemplified herein, one of skill in the art will appreciate the following organisms can be employed to make the corresponding fermentation product. For example, lysine can be made with a bacterium selected from the group *coryneform* bacteria, *Entobacteriaceae* and *Bacillus,* as well as *Corynebacterium glutamicum, Corynebacterium efficiens, Corynebacterium callunae, Corynebacterium thermoaminogenes, Corynebacterium ammoniagenes,* and *E. Coli.* MSG can be made with *Corynebacterium Glutamicum* or *E. Coli.* Lactic acid can be made with a Bacteria based process, including Bacillus Smithii, Bacillus Coagulans, *Bacillus Thermoamylovorans, Geobacilus stearothemophilis,* as well as a Yeast based process, including *Candida, Saccharomyces, Shizosaccharomyes, Kluyveromyces, Pichia, Issachenkia or Hansenula.*

### Detailed Description of the Invention

### Materials

### Microorganism for L-Lysine fermentation

Examples of fermenting organisms include *Brevibacterium flavum* 20879 and *Corynebacterium glutamicum* 10178, purchased from CICC (China Center of Industrial Culture Collection).

### Microorganism for L-Glutamic acid fermentation

Example of fermenting organisms include *Corynebacterium glutamicum sp* 10238, purchased from CICC (China Center of Industrial Culture Collection).

### Microorganism for L-Lactic acid fermentation

Example of fermenting organisms include *Lactobacillus rhamnosus,* purchased from CGMCC (China General Microbiological Culture Collection Center).

### Microorganism culture condition

### Agar seed preparing

Lysine fermentation microorganism agar plate medium for use in this invention includes: glucose 5g/L, NaCl: 5g/L, beef extract 10g/L, peptone 10g/L, agar 20g/L, pH 7.0;

Glutamic acid microorganism agar plate medium for use in this invention includes: glucose 5g/L, NaCl: 5g/L, beef extract 10g/L, peptone 10g/L, agar 20g/L, pH 7.0;

Lactic acid microorganism agar plate medium for use in this invention includes: Casein 10.0 g, Beef extract 10.0 g, Yeast extract 5.0 g, Glucose 5.0 g, Sodium acetate 5.0 g, Diammonium citrate 2.0 g, Tween 80 1.0 g, K₂HPO₄ 2.0 g, MgSO₄•7H₂O 0.2 g, MnSO₄•H₂O 0.05 g, agar 20g, Distilled water 1.0L, pH6.8.

The agar plate medium is sterilized at 121°C holding for 20min(vertical heating pressure steam sterilizer,LDZM-80KCS,Shanghai Shenan medical instrument factory) then dispense to plate, after solidification, cultivate in room temperature for 24hrs for contamination test. The bacterial is transferred into agar plate at clean bench (OptiMair®,Ecso Laminar Flow cabinet) and cultivate in BINDER incubator 24hrs.

### Fermentation seed preparing

Lysine fermentation microorganism seed medium for use in this invention includes: Glucose 25 g/L, CSLP (Corn Steep Liquor Powder) 24 g/L, (NH₄)₂SO₄ 5 g/L, KH₂PO₄ 1g/L, MgSO₄•7H₂O 0.5 g/L, CaCO₃ 15 g/L, pH 7.0. Prepare 1L seed medium, adjust pH according to the Prescription above, then dispense 60mL to every 500mL baffled flask for better oxygen dissolve, then sterilized at 121°C holding for 20min, after cool down to room temperature, transfer the biomass from agar plate to flasks, then cultivate on a NBS rotary shaker at 130 rpm, 30°C, 8∼10hrs, maintain the OD above 0.8, check the strain through microscope to make sure the bacterial shape was "v-shape" and without contamination.

Glutamic acid fermentation microorganism seed medium for use in this invention includes: Glucose 25 g/L, CSLP 24 g/L, K₂HPO₄ 1.5g/L, MgSO₄•7H₂O 0.4 g/L, urea 5 g/L, pH 7.3. Prepare 1L seed medium, adjust pH according to the Prescription above, then dispense 60mL to every 500mL baffled flask for better oxygen dissolve, then sterilized at 121°C holding for 20min, after cool down to room temperature, transfer the biomass from agar plate to flasks, then cultivate on a NBS rotary shaker at 130 rpm, 32°C, 5∼6hrs, maintain the OD above 0.9, check the strain through microscope to make sure the bacterial shape was "v-shape" and without contamination.

Lactic acid fermentation microorganism seed medium for use in this invention includes: Casein 10.0 g, Beef extract 10.0 g, Yeast extract 5.0 g, Glucose 5.0 g, Sodium acetate 5.0 g, Diammonium citrate 2.0 g, Tween 80 1.0 g, K₂HPO₄ 2.0 g, MgSO₄•7H₂O 0.2 g, MnSO₄•H₂O 0.05 g, pH6.8. Prepare 1L seed medium, adjust pH according to the Prescription above, then dispense 100mL to every 300mL flask, then sterilized at 121°C holding for 20min, after cool down to room temperature, transfer the biomass from agar plate to flasks, then cultivate on a NBS rotary shaker at 150 rpm, 37°C, 14∼18hrs, maintain the OD above 0.5, check the strain through microscope to make sure no contamination.

### Fermentation medium preparing

Lysine fermentation microorganism fermentation nutrient medium in 7L jar fermentor includes: Initial glucose 80 g/L,CSLP 15 g/L, (NH₄)₂SO₄ 15g/L, KH₂PO₄ 1g/L, MgSO₄•7H₂O 0.5 g/L, FeSO₄•7H₂O 9.9mg/L, MnSO₄•H₂O6.15mg/L, Vitamin B1(VB1) 100 µg/L, Biotin(VH) 100 µg/L, Antifoam THIX-298 0.05g/L. For lysine fermentation the initial working volume is 3.5L (nutrition 2.75L, initial glucose 0.4L, seed broth 0.35L), nutrition prepare: weight powder CSL (Corn Steep Liquor) 52.5g, (NH4)₂SO₄ 52.5g, KH₂PO₄ 3.5g, MgSO₄•7H₂O 1.75g, FeSO₄•7H₂O 7mg, MnSO₄•H₂O 7mg, VB₁ 350µg, VH 350µg, antifoam 0.175g, dissolved in 2.75L water added to fermentor. Initial glucose prepare: weight 240g glucose dissolved in water and make the total volume at 400mL and add in one flask. High concentration glucose prepare (for feeding in the fermentation process): weight 400g glucose dissolved in water and make the total volume at 500mL and add in one flask using a pipe to gear into the fermentor. Antifoam prepare: weight 20g antifoam dissolved in 80g water and then add in one flask using a pipe to gear into the fermentor. Prepare an empty flask (used for NH₃•H₂O in the fermentation process to adjust pH) and using a pipe to gear into the fermentor, Calibrate pH electrode (METTLER TOLEDO,InPro®3030) and polarize dissolved oxygen electrode(METTLER TOLEDO) up 8hrs,Fix the fermentor, make sure all the flasks stay along with the fermentor, and all the medium should be sterilized at 121°C holding for 20 min,Repeat all the things above for another 7L fermentor fermentation medium prepare. After sterilization, the fermentor should be pump into sterile air to avoid contamination; Pump the initial glucose to fermentor, and add NH₃•H₂O to the empty flask quickly, then adjust the pH to 6.5; Open the water tap, and control the temperature at 30C by auto. Set up the stir at 200rpm, waiting for about half an hour for all the parameters moderation, set the DO electrode at 100%;350mL seed broth added to the fermentor, one fermentor with enzyme and one fermentor without enzyme, begin fermentation, after seed add to fermentor the DO will decrease sharply, the agitation speed and aeration should be increased to maintain the DO at 15%∼30%; In the fermentation process, samples were withdrawn every three or four hours to measure glucose and lysine, if the glucose concentration below 30g/L, then begin feed high concentration of glucose to maintain the glucose concentration around 30g/L∼40g/L.

Glutamic acid fermentation microorganism fermentation nutrient medium in 7L jar fermentor includes: Initial glucose 90-100 g/L,CSLP 4 g/L, MgSO₄•7H₂O 0.8 g/L,, K₂HPO₄ 2g/L,FeSO₄•7H₂O 22mg/L, MnSO₄•H₂O 22 mg/L, Vitamin B₁(VB₁) 0.2mg/L, antifoam THIX-298 0. 1g/L. For glutamic acid fermentation the initial working volume can be 2.5∼2.7L, feeding glucose can be 200ml∼500ml. For example if glutamic acid fermentation the initial working volume is 2.5L (nutrition 1.8L, initial glucose 0.4L, seed broth 0.30L), nutrition prepare: weight powder CSL 10.0g, MgSO₄•7H₂O 2.0 g, KH₂PO₄ 5g, FeSO₄•7H₂O 55mg, MnSO₄•H₂O 55mg, VB₁ 500µg, antifoam 0.25g, dissolved in 1.8L water added to fermentor. Initial glucose prepare: weight 250g glucose dissolved in water and make the total volume at 400mL and add in one flask. High concentration glucose prepare 770g/L (for feeding in the fermentation process): weight 385g glucose dissolved in water and make the total volume at 500mL and add in one flask using a pipe to gear into the fermentor. Antifoam prepare: weight 40g antifoam dissolved in 160g water and then add in one flask using a pipe to gear into the fermentor. Prepare an empty flask (used for NH₃•H₂O in the fermentation process to adjust pH) and using a pipe to gear into the fermentor, Calibrate pH electrode (METTLER TOLEDO,InPro®3030) and polarize dissolved oxygen electrode(METTLER TOLEDO) up 8hrs,Fix the fermentor, make sure all the flasks stay along with the fermentor, and all the medium should be sterilized at 121°C holding for 20min,Repeat all the things above for another 7L fermentor fermentation medium prepare. After sterilization, the fermentor should be pumped into sterile air to avoid contamination; Pump the initial glucose to fermentor, and add NH₃•H₂O to the empty flask quickly, then adjust the pH to 7.0-7.1; Open the water tap, and control the temperature at 30°C by auto. Set up the stir at 200rpm, waiting for about half an hour for all the parameters moderation, set the DO electrode at 100%;300mL seed broth added to the fermentor, one fermentor with enzyme and one fermentor without enzyme, begin fermentation, after seed add to fermentor the DO will decrease sharply, the agitation speed and aeration should be increased to maintain the DO at 30%∼50%; In the fermentation process, after 4∼6hrs increase fermentation temperature 1°C every three or four hours till 38°C, samples were withdrawn every three or four hours to measure glucose and glutamic acid, if the glucose concentration below 20g/L, then begin feed high concentration of glucose to maintain the glucose concentration around 20g/L∼30g/L.

Lactic acid fermentation microorganism fermentation nutrient medium in 1L jar fermentor includes: Initial sugar 80 g/L,CSLP 40 g/L, casein 10 g/L, beef extract 10 g/L, yeast extract 10g/L, Tween 80 1.5 g/L, MnS0₄·H₂O 0.3g/L, calcium carbonate 20g/, pH 6.5. For Lactic acid fermentation the initial working volume is 0.6L (nutrition 0.5L, initial glucose 0.05L, seed broth 0.05∼0.10L), nutrition prepare: weight powder corn steep liquor 20 g, casein 5.0 g, Beef extract 5.0 g, Yeast extract 5.0 g, Tween 80 0.75 g, MnSO4•H2O 0.15 g, Calcium carbonate 10g,pH 6.5,Initial glucose prepare: weight 40g glucose dissolved in water and make the total volume at 50mL and add in one flask. High concentration glucose prepare (for feeding in the fermentation process): weight 100g glucose dissolved in water and make the total volume at 150mL and add in one flask using a pipe to gear into the fermentor. Prepare an empty or contained 50ml distilled water flask (used for NH₃•H₂O in the fermentation process to adjust pH,) and using a pipe to gear into the fermentor, Calibrate pH electrode (METTLER TOLEDO,InPro®3030) and polarize dissolved oxygen electrode(METTLER TOLEDO) up 8hrs,Fix the fermentor, make sure all the flasks stay along with the fermentor, and all the medium should be sterilized at 121°C holding for 20min,Repeat all the things above for another 1L fermentor fermentation medium prepare. After sterilization, the fermentor should be pump into sterile air to avoid contamination; Pump the initial glucose to fermentor, and add NH₃•H₂O to the empty flask quickly, then adjust the pH to 6.5; Open the water tap, and control the temperature at 40∼45°C by auto. Set up the stir at 200rpm, waiting for about half an hour for all the parameters moderation, then added 50mL seed broth to the fermentor, one fermentor with enzyme and one fermentor without enzyme, begin fermentation, In the fermentation process, pH was adjusted automatically by adding additional NH₃•H₂O or diluted NH₃•H₂O. Samples were withdrawn every three or four hours to measure sugar and lactic acid level analyzed by HPLC.

As used herein, CSLP refers to the spray-dried Corn Steep Liquor Powder, which is widely used in Microbial Fermentation, purchased from Roquette Co., Ltd. The glucose syrup is Liquid Glucose purchased from Xiwang Group Co.,Ltd. HPAED analysis results indicated greater than 85% glucose expressed as mg/L. AntifoamTHIX-298 was bought from YanTai Thinking Finechem Technology Co. Ltd in Shandong province. The 7L jar fermentor was purchased from KOBIOTECH CO., LTD. The 1L jar fermentor was purchased from APPLITECH CO., LTD. Unless otherwise specified, other chemicals are purchased from Sinopharm Co., Ltd; beef extract, peptone, casein, agar are BR grade, others are AR grade.

### Enzymes

Transglucosidase L-2000(product of DuPont Industrial Biosciences, 2013): purified D-glucosyltransferase (transglucosidase, EC 2.4.1.24) free from glucoamylase activity. Transglucosidase L-2000 is produced through controlled fermentation, using a genetically modified strain of *Trichoderma reesei.*

Transglucosidase L "Amano"(manufactured by Amano Pharmaceutical Co., Ltd.),): the *Aspergillus niger* glucosyltransferase. This enzyme is an α-glucosidase (EC 3.2.1.20), and is capable of hydrolyzing its substrate from the non-reducing end of the molecule to release glucose molecules. Also, under high substrate concentration, this enzyme is capable of performing a glycosyl transfer reaction rather than the hydrolysis of the substrate. Because of this glycosyl transfer capability, this enzyme is also referred as transglucosidase.

Optimash® BG(Genencor International, Inc): Viscosity Reducing Enzyme, High activity betaglucanase, xylanase and cellulase enzyme complex for rapid viscosity reduction, made in a modified Trichoderma host in which CBH1 and CBH2 cellobiohydrolases I and II) are deleted, and EGII (endoglucanase, a beta glucanase) is over-expressed by at least 50% relative to wild type, as taught for example in WO2A118257.

TrGA (Genencor International, Inc): Glucoamylase enzyme produced by a non-pathogenic, non-toxigenic strain of *Trichoderma reesei,* which is genetically modified to over express a native *T.reesei* glucoamylase enzyme (see for example U.S. Patent 7,413,887)

ACCELLERASE ® DUET (Genencor International, Inc): produced with a genetically modified strain of Trichoderma *reesei,* that contains not only exoglucanase, endoglucanase, β-glucosidase, but also includes xylanases and other hemicellulases, as commercially available from Danisco US Inc, DuPont Industrial Biosciences, in 2013.

### Methods

### Sugar content determination by HPAED

Sugar analysis was performed on a Dionex ICS-5000 ion chromatography (Sunnyvale, CA, USA) composed of a GP50 gradient pump, an ED 40 electrochemical detector including a pulsed amperometric detection cell made of a gold electrode and a pH-Ag/AgCl reference electrode. Separation was performed on a CarboPakTM PA200 column (3*250mm) with a guard column (3*50mm). The flow rate was 0.5 ml/min on CarboPakTM PA200 column, and column temperature was 30°C. Sample injection volume was 25 µl. The gradient for CarboPakTM PA200 column used was: 0-5min, 100mM NaOH, 0-40mM NaAc; 5-60 min, 100mM NaOH, 40-500mM NaAc; 60-70min, 100mM NaOH. Pulsed amperometric detection was used as detector, and following pulse potentials and durations were used: E1 = 0.1V, t1 = 400 ms; E2 = -2V, t2 = 20 ms; E3 = 0.6V, t3 = 10 ms; E4 = -0.1V, t4 = 70 ms. Data acquisition and integration were performed using Chromeleon 6.8 workstation.

### Fermentation biomass determination by Spectrophotometer

Bio-mass analysis was performed on SHIMADZU UV-1700 Spectrophotometer. Fermentation broth was diluted 25 times with 1mol/L Hydrochloric acid, and then checked the OD (Optical Density) value at 562nm for lysine or 620nm for glutamic acid.

### Residual glucose and lysine / Glutamic acid content determination by SBA40C Bio-sensor

Both residual glucose and lysine yield were performed on SBA-40C bio-sensor (Shandong Academy of Sciences Institute of Biology). The fermentation broth was diluted 100 times with purified water, before analyzing sample calibration was required. Injected 25 µl standard sample (100mg/dL glucose and 100mg/dl lysine stock solution) several times to complete the calibration. Injected 25 µl sample and record the data showed on the screen. Each time of calibration 10 samples can be analyzed. If the sample number was more than 10, recalibration step was performed.

### Lactic acid content determination by HPLC(High Pressure Liquid Chromatographic):

HPLC method using an Agilent 1100, Column specification: BIO-RAD Aminex HPX-87H or Rezex ROA-organic acid. Method of analysis: ESTD. Details of the analysis: Mobile phase: 0.005 mol/L H₂SO₄. Sample was withdrawn and diluted 10 times, and filtered using 0.45µm filter membrane. Other HPLC parameters: Injection volume: 20 µl; Pump flow: 0.6 ml/min; Column thermostat temperature: 60°C; RID, optical unit temperature: 35°C.

### Fermentation broth viscosity determination by HAAKE VT550

Viscosity analysis was performed on HAAKE VT550 viscometer with FL-10 spindle in DC30-K10 Refrigerated Circulator water bath (all from Thermo Scientific). Weighed out 100 grams fermentation broth into HAAKE tube, using FL-10 spindle and run fixed share rate Á 10 [1/s] fixed temperature 30°C process 10min. When the process was terminated, poured the broth and cleaned the tube to run another batch. Viscosity data were recorded by HAAKE workstation.

### Natural insoluble solids settling observation

Poured the fermentation broth into a beaker and covered it with wrap, hold it in 4-8°C refrigerator 18hrs, and then took a picture.

### Example 1 :

### Comparison of commercial Transglucosidase activity containing products on lysine fermentation using sugar syrup as substrate: Brevibacterium flavum

Bacterial biomass collected from an agar plate transferred into seed culture medium in 500 mL flasks. Cultivate on a NBS rotary shaker at 130 rpm and 30 °C, and the working volume 60 mL/500mL flask, using baffled flask, cultivate about 10 hrs and maintain the OD above 0.8. The fermentation medium was sterilized at 121°C for 12 min, cooled down the temperature to 30°C, add 80g/L initial glucose to the fermentor, adjust the pH of the medium to 6.5 by ammonium hydroxide. 0.75g TrTG L-2000(product of Genencor International, Inc.) and Transglucosidase ™ L "Amano"(manufactured by Amano Pharmaceutical Co., Ltd., Japan) with 300 mL seed broth (10% v/v) was added to fermentation medium. Temperature was maintained at 30°C, and pH was controlled at 6.8∼7.0 by addition of ammonium hydroxide, DO was maintained at 10%∼25% by adjusting aeration and agitation speed. In the fermentation process, samples were withdrawn every three or four hours to measure glucose and maintain at suitable concentration (30g/L∼40g/L) by feeding high concentration of glucose, at 8hrs and 32hrs added 0.75g TrTG and AMANO TG respectively.

**Table 1: Effect of Transglucosidase on lysine fermentation yield, residual glucose at the end of fermentation and viscosity of fermentation broth using glucose syrup as substrate**

| | Viscosity (mpas) | | Residual glucose (g/L) | | Lysine•HCl (g/L) | |
|---|---|---|---|---|---|---|
| | BK | With Enzyme | BK | With Enzyme | BK | With Enzyme |
| TrTG@ 3.8Kg/MT | 70.26 | 21.62 | 11 | 2 | 51.3 | 68.8 |
| AMANO TG @3.6Kg/MT | 54.04 | 81.07 | 16 | 2 | 37.5 | 52.5 |

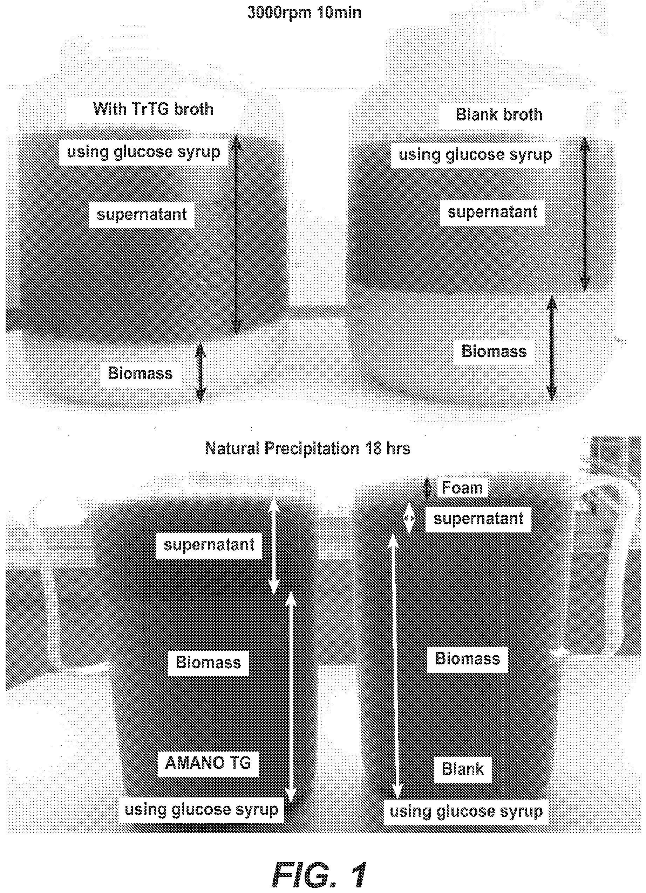

The results of Table 1 and Figure 1 showed that additional TrTG L2000 resulted in much higher lysine. HCl yield , lower residual glucose, lower broth viscosity, and faster precipitation than blank (BK). Additionally, AMANO TG resulted in much higher lysine. HCl yield, lower residual glucose, and faster precipitation than blank as TrTG L2000 showed, but resulted in much higher broth viscosity than blank.

### Example 2

### TrTG L2000 effect on lysine fermentation using pure glucose as substrate: Brevibacterium flavum

Bacterial biomass collected from an agar plate transferred into seed culture medium in 500 mL flasks. Cultivate on a NBS rotary shaker at 130 rpm and 30°C, and the working volume 60 mL/500mL flask, using baffled flask, cultivate about 10 hrs and maintain the OD above 0.8. The fermentation medium was sterilized at 121°C for 12 min, cooled down the temperature to 30°C, add 80g/L initial glucose to the fermentor, adjust the pH of the medium to 6.5 by ammonium hydroxide. 0.75g TrTG L-2000 (product of Genencor International, Inc.) and with 300 mL seed broth (10% v/v) was added to fermentation medium. Temperature was maintained at 30°C, and pH was controlled at 6.8∼7.0 by addition of ammonium hydroxide, DO was maintained at 10%∼25% by adjusting aeration and agitation speed. In the fermentation process, samples were withdrawn every three or four hours to measure glucose and maintain at suitable concentration (30g/L∼40g/L) by feeding high concentration of glucose, at 8hrs and 32hrs added 0.75g TrTG L2000 respectively.

**Table 2: Effect of TrTG L 2000 on lysine fermentation yield, residual glucose at the end of fermentation and viscosity of fermentation broth using pure glucose as substrate**

| | Viscosity (mpas) | | Residual glucose (g/L) | | Lysine•HCl (g/L) | |
|---|---|---|---|---|---|---|
| | BK | With Enzyme | BK | With Enzyme | BK | With Enzyme |
| TrTG @3.7Kg/MT | 54.04 | 21.62 | 1 | 1 | 37.5 | 46.3 |

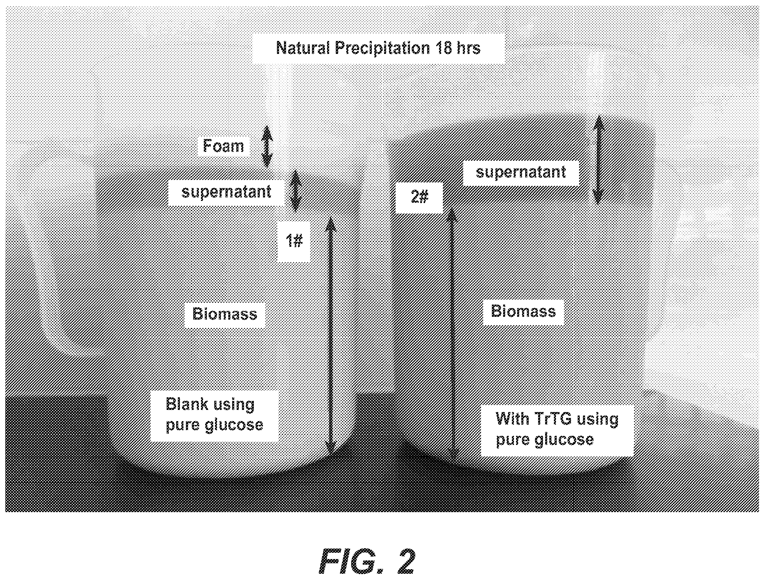

Table 2 and Figure 2 showed that additional TrTG L2000 resulted in much higher lysine·HCl yield, lower residual glucose, lower broth viscosity, and faster precipitation than blank.

### Example 3 Effect of TrGA on Lysine production using sugar syrup as substrate: Brevibacterium flavum

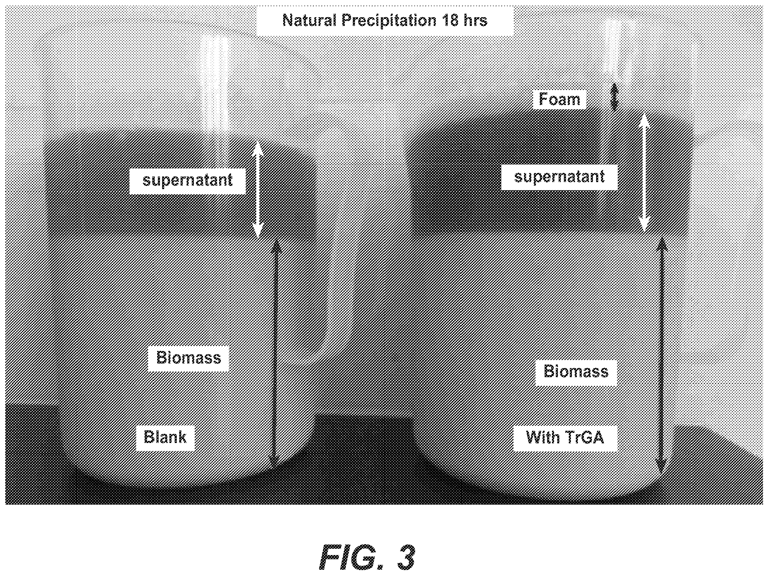

This experiment assessed whether TG was likely responsible for the improvements, or if residue from the Trichoderma production strain is likely responsible. Bacterial biomass collected from an agar plate transferred into seed culture medium in 500 mL flasks. Cultivate on a NBS rotary shaker at 130 rpm and 30°C, and the working volume 60 mL/500mL flask, using baffled flask, cultivate about 10 hrs and maintain the OD above 0.8. The fermentation medium was sterilized at 121°C for 12 min, cooled down the temperature to 30°C, add 80g/L initial glucose to the fermentor, adjust the pH of the medium to 6.5 by ammonium hydroxide. Controlled fermentation pH at 6.8∼7.0 by addition of ammonium hydroxide, DO was maintained at 10%∼25% by adjusting aeration and agitation speed. In the fermentation process, samples were withdrawn every three or four hours to measure glucose and maintain at suitable concentration (30g/L∼40g/L) by feeding glucose syrup(500g/400ml). TrGA was added at the beginning of the fermentation with 0.715g and 8h with 0.715g, so the total TrGA was 2.4Kg/MT ds glucose. After fermentation, check the Lysine production, residual sugar, broth viscosity and precipitation (Table.3, Fig.3)

**Table 3: Effect of TrGA on lysine fermentation yield, residual glucose at the end of fermentation and viscosity of fermentation broth using glucose syrup as substrate**

| | Viscosity (mpas) | | Residual glucose (g/L) | | Lysine•HCl (g/L) | |
|---|---|---|---|---|---|---|
| | BK | With Enzyme | BK | With Enzyme | BK | With Enzyme |
| TrGA @ 2.4kg/MT | 37.83 | 54.04 | 5 | 10 | 52.5 | 46.3 |

The results showed that additional TrGA during lysine fermentation increased the broth viscosity and residual glucose but decreased Lysine·HCl production. The decrease in lysine production is indicated by comparison to the blank, which is lower. Without being bound by theory, this observation supported the hypothesis that TrTG is responsible for the improved lysine fermentation, and that residue from the Trichoderma expression host may not be responsible.

### Example 4 Effect of OPTIMASH ™ BG on Lysine production using pure glucose as substrate: Brevibacterium flavum

Bacterial biomass collected from an agar plate transferred into seed culture medium in 500 mL flasks. Cultivate on a NBS rotary shaker at 130 rpm and 30°C, and the working volume 60 mL/500mL flask, using baffled flask, cultivate about 10 hrs and maintain the OD above 0.8. The fermentation medium was sterilized at 121°C for 12 min, cooled down the temperature to 30°C, the initial glucose was 80g/L, controlled pH at 6.8∼7.0 by addition of ammonium hydroxide, DO was maintained at 10%∼25% by adjusting aeration and agitation speed. In the fermentation process, samples were withdrawn every three or four hours to measure glucose and maintain at suitable concentration (30g/L∼40g/L) by feeding glucose solution (500g/400ml).OPTIMASH BG was added at the beginning of the fermentation, 16h, and 32h with 1.12g, 0.8g, 0.8g separately, the total dosage of OPTIMASH BG addition was 4Kg/MT ds glucose, after fermentation, checked the Lysine production, residual sugar and broth viscosity.

**Table 4: Effect of OPTIMASH BG on lysine fermentation yield, residual glucose at the end of fermentation and viscosity of fermentation broth using pure glucose as substrate**

| | Viscosity (mpas) Residual glucose (g/L) Lysine•HCl (g/L) | | | | | |
|---|---|---|---|---|---|---|
| | BK | With Enzyme | BK | With Enzyme | BK | With Enzyme |
| OPTIMASH BG @ 4.0kg/MT | 31.00 | 28.90 | 25 | 4 | 32.5 | 43.8 |

The results showed that additional OPTIMASH BG during lysine fermentation potentially slightly decreased broth viscosity and residual glucose, and significantly increased Lysine·HCl production.

### Example 5 Effect of Transglucosidase on Lysine production using pure glucose as substrate: Corynebacterium glutamicum 10178

Bacterial biomass collected from an agar plate transferred into seed culture medium in 500 mL flasks. Cultivate on a NBS rotary shaker at 130 rpm and 30 °C, and the working volume 60 mL/500mL flask, using baffled flask, cultivate about 10 hrs and maintain the OD above 0.8. The fermentation medium was sterilized at 121°C for 12 min, cooled down the temperature to 30°C, the initial glucose was 100g/L, controlled pH at 6.8∼7.0 by addition of ammonium hydroxide, DO was maintained at 10%∼25% by adjusting aeration and agitation speed. In the fermentation process, samples were withdrawn every three or four hours to measure glucose and maintain at suitable concentration (30g/L∼40g/L) by feeding glucose solution (800g/1000ml). Transglucosidase L-2000(product of Genencor International, Inc.) was added at the beginning of the fermentation, 15h, 23h, 32h, and 36h with 1.4g, 0.8g, 0.8g, 0.8g and 0.8g separately. The total dosage of Transglucosidase L-2000 was 4Kg/MT ds glucose, after fermentation, checked the Lysine production, residual sugar, broth viscosity and broth color.

**Table 5: Effect of TrTG on lysine fermentation yield, residual glucose at the end of fermentation and viscosity of fermentation broth using pure glucose as substrate, strain Corynebacterium glutamicum 10178.**

| | Viscosity (mpas) | | Residual glucose (g/L) | | Lysine•HCl (g/L) | |
|---|---|---|---|---|---|---|
| | BK | With Enzyme | BK | With Enzyme | BK | With Enzyme |
| TrTG @ 4.0kg/MT | 6.50 | 7.42 | 11 | 16 | 57.5 | 55.0 |

The results showed that although the color changed with additional Transglucosidase L-2000, however, the enzyme did not affect the Lysine·HCl production.

### Example 6 Effect of Transglucosidase on Glutamic acid production using pure glucose as substrate: Corynebacterium glutamicum SP 10238

Bacterial biomass collected from an agar plate transferred into seed culture medium in 500 mL flasks. Cultivate on a NBS rotary shaker at 130 rpm and 32 °C, and the working volume 60 mL/500mL flask, using baffled flask, cultivate about 5-6 hrs and maintain the OD above 0.9. The fermentation medium was sterilized at 121°C for 12 min, cooled down the temperature to 30°C. The initial glucose was 100g/L, controlled pH at 7.0∼7.1 by addition of ammonium hydroxide, DO was maintained at 20%∼40% by adjusting aeration and agitation speed. In the fermentation process, after 4∼6hrs increase fermentation temperature 1°C every three or four hours till 38°C, samples were withdrawn every three or four hours to measure glucose and glutamic acid, if the glucose concentration below 20g/L, then begin feed high concentration of glucose to maintain the glucose concentration around 20g/L∼30g/L. Transglucosidase L-2000(product of Genencor International, Inc.) was added at the beginning of the fermentation(Oh), 8h, 16h, 21h with0.9525g, 0.9525g, 0.3175g, and 0.3175g separately. The total dosage of Transglucosidase L-2000 was 4Kg/MT ds glucose, took picture during fermentation to record foaming level changing, record the glutamic acid production, residual sugar, antifoam consumption, broth viscosity and broth color after fermentation.

**Table 6: Effect of TrTG on Glutamic acid fermentation yield, residual glucose at the end of fermentation and viscosity of fermentation broth using pure glucose as substrate, strain Corynebacterium glutamicum SP 10238.**

| | Viscosity (mpas) | | Residual glucose (g/L) | | Glutamic acid (g/L) | |
|---|---|---|---|---|---|---|
| | BK | With Enzyme | BK | With Enzyme | BK | With Enzyme |
| TrTG@4Kg/MT | 21.62 | 5.4 | 23 | 7 | 59 | 63 |

**Table 7: Effect of TrTG on antifoam consumption using pure glucose as substrate, strain Corynebacterium glutamicum SP 10238.**

| Time(hrs) | With TrTG broth height (cm) | Blank broth height (cm) | With TrTG Foam height(cm) | Blank Foam height(cm) |
|---|---|---|---|---|
| 0 | 13 | 13 | 0 | 0 |
| 4 | 13 | 13 | 0 | 0 |
| 6 | 13 | 13 | 0 | 3 |
| 12 | 13 | 13 | 2 | 4-5 |
| 13 | 13 | 13 | 2 | 8 (begin to add antifoam) |
| 15 | 13 | 13 | 3 | 15 |
| 21 | 14 | 14 | 7 (begin to add antifoam) | 15 |
| 45hrs total 20% antifoam consumption (ml) | | | 40 | 100 |

These results showed that additional TrTG during glutamic acid fermentation decreased broth viscosity and residual glucose and significantly increased glutamic acid production, and at same time reduced the foaming formation and decreased antifoam consumption at least 50%.

### Example 7 Effect of ACCELLERASE ™ DUET on Glutamic acid production using pure glucose as substrate: Corynebacterium glutamicum SP 10238

Bacterial biomass collected from an agar plate transferred into seed culture medium in 500 mL flasks. Cultivate on a NBS rotary shaker at 130 rpm and 32°C, and the working volume 60 mL/500mL flask, using baffled flask, cultivate about 5-6 hrs and maintain the OD above 0.9. The fermentation medium was sterilized at 121°C for 12 min, cooled down the temperature to 30°C, the initial glucose was 92.5g/L, controlled pH at 7.0∼7.1 by addition of ammonium hydroxide, DO was maintained at 30%∼50% by adjusting aeration and agitation speed. In the fermentation process, after 4∼6hrs increase fermentation temperature 1°C every three or four hours till 38°C, samples were withdrawn every three or four hours to measure glucose and glutamic acid, if the glucose concentration below 20g/L, then begin feed high concentration of glucose to maintain the glucose concentration around 20g/L∼30g/L.ACCELLERASE DUET (product of DuPont Industrial Biosciences, 2013) was added at the beginning of the fermentation(Oh), 8h, 16h, with 0.808g, 0.808g, and 0.202g separately. The total dosage of ACCELLERASE DUET was 4.5Kg/MT ds glucose, took picture during fermentation to record foaming level changing, record the glutamic acid production, residual sugar, antifoam consumption, broth viscosity and broth color after fermentation.

**Table 8: Effect of ACCELLERASE ™ DUET on Glutamic acid fermentation yield, residual glucose at the end of fermentation and viscosity of fermentation broth using pure glucose as substrate, strain Corynebacterium glutamicum SP 10238.**

| | Viscosity (mpas) | | Residual glucose (g/L) | | Glutamic acid (g/L) | |
|---|---|---|---|---|---|---|
| | BK | With Enzyme | BK | With Enzyme | BK | With Enzyme |
| ACCELLERASE DUET @ 4.5kg/MT | 27.02 | 5.4 | 7 | 3 | 47 | 50 |

**Table 9: Effect of ACCELLERASE on antifoam consumption using pure glucose as substrate, strain Corynebacterium glutamicum SP 10238.**

| | 20% antifoam (ml) |
|---|---|
| Blank | 130 |
| Accellerase DUET @ 4.5kg/MT | 100 |

The results showed that additional ACCELLERASE DUET during glutamic acid fermentation significantly decreased broth viscosity, slightly decreased residual glucose and slightly increased glutamic acid production, and at the same time reduced the foaming formation and decreased antifoam consumption 30%.

### Example 8: Effect of Transglucosidase on Lactic acid production using pure sucrose as substrate: Lactobacillus rhamnosus

Bacterial biomass collected from an agar plate transferred into seed culture medium in 300 mL flasks. Cultivate on a NBS rotary shaker at 150 rpm and 37°C, and the working volume 100 mL/300mL flask, cultivate about 14-18 hrs and maintain the OD above 0.5. The fermentation medium was sterilized at 121°C for 12 min, cooled down the temperature to 40°C, the initial sucrose was 80g/L (40g/500ml), controlled pH at 6.5 by addition of ammonium hydroxide, samples were withdrawn every three or four hours to measure sucrose and lactic acid, from 20hrs to 32hrs feed high concentration of sucrose 150ml (100g/150m). TrTG was added at the beginning of the fermentation(Oh), 20h, 21h, with 0.145g, 0.145g, and 0.140g separately. The total dosage of TrTG was 3.07Kg/MT ds sucrose, analyzed Lactic acid production, broth viscosity after fermentation.

**Table 10: Effect of TrTG on Lactic acid fermentation yield, residual sucrose at the end of fermentation and viscosity of fermentation broth using pure sucrose as substrate, strain Lactobacillus rhamnosus**

| | Fermentation time (hrs) | Total sucrose (g) | Lactic acid (g/L) | NH3.H2O volume (ml) | Final volume (ml) | Lactic acid yield (%) | Residual sucrose (g/L) | Viscosity (mpas) |
|---|---|---|---|---|---|---|---|---|
| Control | 49 | 140 | 20 | 10 | 660 | 14.29 | / | 28.21 |
| TrTG @ 3.07kg/MT | 49 | 140 | 31 | 10 | 660 | 22.14 | / | 16.21 |

The results showed that additional TrTG during lactic acid fermentation significantly decreased broth viscosity and increased lactic acid production.

### Example 9: Effect of Transglucosidase on Lactic acid production using pure glucose as substrate: Lactobacillus rhamnosus

Bacterial biomass collected from an agar plate transferred into seed culture medium in 300 mL flasks. Cultivate on a NBS rotary shaker at 150 rpm and 37°C, and the working volume 100 mL/300mL flask, cultivate about 14-18 hrs and maintain the OD above 0.5. The fermentation medium was sterilized at 121°C for 12 min, cooled down the temperature to 40°C, the initial glucose was 80g/L (40g/500ml), controlled pH at 6.5 by addition of ammonium hydroxide, samples were withdrawn every three or four hours to measure sucrose and lactic acid, from 4hrs to 22hrs feed high concentration of glucose 150ml (100g/150m). TrTG was added at the beginning of the fermentation (0h), 10h, 22h, with 0.150g, 0.150g, and 0.300g separately. The total dosage of TrTG was 4.28Kg/MT ds glucose, analyzed Lactic acid production, residual sugar, broth viscosity after fermentation.

**Table 11: effect of TrTG on Lactic acid fermentation yield, residual glucose at the end of fermentation and viscosity of fermentation broth using pure glucose as substrate, strain Lactobacillus rhamnosus**

| | Fermentation time (hrs) | Total glucose (g) | Lactic acid (g/L) | Diluted NH3.H2O volume (ml) | Total volume (ml) | Lactic acid yield (%) | Residual glucose (g/L) | Viscosity (mpas) |
|---|---|---|---|---|---|---|---|---|
| Control TrTG @ 4.28 kg/MT | 71 | 140 | 97.4 | 100 | 750 | 52.09 | 64.2 | 15.72 |
| | 71 | 140 | 103.6 | 230 | 880 | 65.16 | 42.3 | 12.97 |

The results showed that additional TrTG during lactic acid fermentation significantly decreased broth viscosity, residual glucose and increased lactic acid production.

### Example 10 Effect of OPTIMASH BG on Lactic acid production using pure glucose as substrate: Lactobacillus rhamnosus

Bacterial biomass collected from an agar plate transferred into seed culture medium in 300 mL flasks. Cultivate on a NBS rotary shaker at 150 rpm and 37°C, and the working volume 100 mL/300mL flask, cultivate about 14-18 hrs and maintain the OD above 0.5. The fermentation medium was sterilized at 121°C for 12 min, cooled down the temperature to 45°C, the initial glucose was 80g/L (40g/500ml), controlled pH at 6.5 by addition of ammonium hydroxide, samples were withdrawn every three or four hours to measure sucrose and lactic acid, from 6hrs to 22hrs feed high concentration of glucose 150ml(100g/150m). OPTIMASH BG was added at the beginning of the fermentation (0h), 22h, with 0.150g, 0.220g separately. The total dosage of OPTIMASH BG was 3.70Kg/MT ds glucose, analyzed Lactic acid production, residual sugar, broth viscosity after fermentation.

**Table 12: effect of OPTIMASH BG on Lactic acid fermentation yield, residual glucose at the end of fermentation and viscosity of fermentation broth using pure glucose as substrate, strain Lactobacillus rhamnosus**

| | Fermentation time (hrs) | Total glucose (g) | LA production(g/L) | Diluted NH3.H2O volume (ml) | Total volume (ml) | Lactic acid yield (%) | Residual glucose (g/L) | Viscosity (mpas) |
|---|---|---|---|---|---|---|---|---|
| Blank OPTIMASH BG @3.7Kp/MT | 46 | 140 | 69.8 | 150 | 800 | 55.84 | 56.9 | 8.10 |
| | 46 | 140 | 108.3 | 190 | 840 | 77.36 | 21.5 | 0.00 |

The results showed that additional OPTIMASH BG during lactic acid fermentation significantly decreased broth viscosity, residual glucose and increased lactic acid production.

## Claims

1. A method for reducing the viscosity and/or foam of a fermentation broth, comprising;
fermenting a feedstock with a fermenting microorganism in a fermentation broth to make a fermentation product, wherein the fermentation broth comprises a transglycosidase. and wherein the fermentation product has a higher yield than a control fermentation lacking the transglycosidase;
wherein the transglycosidase has at least 80% amino acid sequence identity to the mature transglucosidase enzyme of SEQ ID NO: 1 and wherein the transglycosidase is produced by expression in a *Trichoderma.*

2. The method of claim 1 further comprising recovering the fermentation product.

3. The method of any of the preceding claims, wherein the fermentation product is lysine, lactic acid, or glutamic acid.

4. The method of any of the preceding claims, wherein the fermenting organism is *Brevibacterium flavum, Corynebacterium glutamicum* 10178, *Corynebacterium glutamicum* 10238, or *Lactobacillus rhamnosus.*

5. The method of any of the preceding claims, wherein the transglycosidase comprises SEQ ID NO: 1 or has at least 85%, 90%, 95%, 98% or 99% amino acid sequence identity to the mature transglucosidase enzyme of SEQ ID NO: 1

6. The method of any of the preceding claims, wherein the feedstock comprises starch liquifact, granular starch, pure glucose, pure sucrose, or sugar syrup.

7. The method of any of the preceding claims, wherein the transglycosidase is a *Trichoderma* transglucosidase (TrTG) and is present at 1-6 Kg/10³ Kg (Kg/MT), 2-5 Kg/10³ Kg (Kg/MT), or 2-4 Kg/10³ Kg (Kg/MT).

8. The method of any one of the preceding claims, wherein:
(a) the fermentation product is lysine and the fermenting organism is *Brevibacterium flavum;* or
(b) the fermentation product is lysine, the fermenting organism is *Brevibacterium flavum* and the transglucosidase is an *A*. *Niger* transglucosidase (TG); or
(c) the fermentation product is glutamic acid and the fermenting organism is *Corynebacterium glutamicum* SP 10238; or
(d) the fermentation product is lactic acid and the fermenting organism is *Lactobacillus rhamnosus.*

9. The method according to claim 8, wherein the feedstock comprises pure glucose or sugar syrup.

10. Use of a transglycosidase in a fermentation to reduce viscosity of a fermentation broth by a fermenting microorganism, wherein the transglycosidase has at least 80% amino acid sequence identity to the mature transglucosidase enzyme of SEQ ID NO: 1.

11. Use of a transglycosidase in a fermentation to reduce foaming of a fermentation broth by a fermenting microorganism, wherein the transglycosidase has at least 80% amino acid sequence identity to the mature transglucosidase enzyme of SEQ ID NO: 1.

12. The use of claim 10 or claim 11, wherein the transglycosidase increases the yield of a fermentation product by a fermenting microorganism.

13. The use according to any one of claims 10 to 12, wherein the transglycosidase has at least 98% amino acid sequence identity to the mature transglucosidase enzyme of SEQ ID NO: 1.

14. The use according to claim 10 to 13, wherein the fermentation makes a fermentation product selected from the group consisting of lysine, citric acid, or glutamic acid.

15. The use according to any of claims 10 to 14, wherein the fermenting microorganism is selected from the group consisting of *Corynebacterium glutamicum* SP 10238, *Lactobacillus rhamnosus, Brevibacterium flavum,* or *Corynebacterium glutamicum* 10178.

## Patentansprüche

1. Verfahren zum Reduzieren der Viskosität und/oder des Schaums einer Fermentationsbouillon, umfassend:
Fermentieren eines Ausgangsmaterials mit einem fermentierenden Mikroorganismus in einer Fermentationsbouillon, um ein Fermentationsprodukt herzustellen, wobei die Fermentationsbouillon eine Transglycosidase umfasst,
und wobei das Fermentationsprodukt eine höhere Ausbeute aufweist als eine Kontrollfermentation, der Transglycosidase fehlt,
wobei die Transglycosidase mindestens 80 % Aminosäuresequenzidentität mit dem ausgereiften Transglycosidaseenzym von SEQ ID NO: 1 aufweist und wobei die Transglycosidase durch Expression in einem *Trichoderma* hergestellt wird.

2. Verfahren nach Anspruch 1, ferner das Gewinnen des Fermentationsprodukts umfassend.

3. Verfahren nach einem der vorhergehenden Ansprüche, wobei das Fermentationsprodukt Lysin, Milchsäure oder Glutaminsäure ist.

4. Verfahren nach einem der vorhergehenden Ansprüche, wobei der Fermentationsorganismus *Brevibacterium flavum, Corynebacterium glutamicum* 10178, *Corynebacterium glutamicum* 10238 oder *Lactobacillus rhamnosus* ist.

5. Verfahren nach einem der vorhergehenden Ansprüche, wobei die Transglycosidase SEQ ID NO: 1 umfasst oder mindestens 85 %, 90 %, 95 %, 98 % oder 99 % Aminosäuresequenzidentität mit dem ausgereiften Transglucosidaseenzym von SEQ ID NO: 1 aufweist.

6. Verfahren nach einem der vorhergehenden Ansprüche, wobei das Ausgangsmaterial eine Stärkeverflüssigung, granuläre Stärke, reine Glucose, reine Sucrose oder Zuckersirup umfasst.

7. Verfahren nach einem der vorhergehenden Ansprüche, wobei die Transglycosidase eine *Trichoderma* Transglucosidase (TrTG) ist und in einer Menge von 1-6 kg/10³kg (kg/t), 2-5 kg/10³ kg (kg/t) oder 2-4 kg/10³ kg (kg/t) vorliegt.

8. Verfahren nach einem der vorhergehenden Ansprüche, wobei:
(a) das Fermentationsprodukt Lysin ist und der fermentierende Organismus *Brevibacterium flavum* ist; oder
(b) das Fermentationsprodukt Lysin ist, der fermentierende Organismus *Brevibacterium flavum* ist und die Transglucosidase eine *A. Niger*-Transglucosidase (TG) ist; oder
(c) das Fermentationsprodukt Glutaminsäure ist und der fermentierende Organismus *Corynebacterium glutamicum* SP 10238 ist; oder
(d) das Fermentationsprodukt Milchsäure ist und der fermentierende Organismus *Lactobacillus rhamnosus* ist.

9. Verfahren nach Anspruch 8, wobei das Ausgangsmaterial reine Glucose oder Zuckersirup umfasst.

10. Verwendung einer Transglycosidase bei einer Fermentation zum Reduzieren der Viskosität einer Fermentationsbouillon durch einen fermentierenden Mikroorganismus, wobei die Transglycosidase mindestens 80 % Aminosäuresequenzidentität mit dem ausgereiften Transglucosidaseenzym von SEQ ID NO: 1 aufweist.

11. Verwendung einer Transglycosidase bei einer Fermentation zum Reduzieren des Schäumens einer Fermentationsbouillon durch einen fermentierenden Mikroorganismus, wobei die Transglycosidase mindestens 80 % Aminosäuresequenzidentität mit dem ausgereiften Transglycosidaseenzym von SEQ ID NO: 1 aufweist.

12. Verwendung nach Anspruch 10 oder Anspruch 11, wobei die Transglycosidase die Ausbeute eines Fermentationsprodukts durch einen fermentierenden Mikroorganismus erhöht.

13. Verwendung nach einem der Ansprüche 10 bis 12, wobei die Transglycosidase mindestens 98 % Aminosäuresequenzidentität mit dem ausgereiften Transglucosidaseenzym von SEQ ID NO: 1 aufweist.

14. Verwendung nach Anspruch 10 bis 13, wobei die Fermentation ein Fermentationsprodukt erzeugt ausgewählt aus der Gruppe bestehend aus Lysin, Zitronensäure oder Glutaminsäure.

15. Verwendung nach einem der Ansprüche 10 bis 14, wobei der fermentierende Mikroorganismus aus der Gruppe ausgewählt wird bestehend aus *Corynebacterium glutamicum* SP 10238, *Lactobacillus rhamnosus, Brevibacterium flavum* oder *Corynebacterium glutamicum* 10178.

## Revendications

1. Procédé pour la réduction de la viscosité et/ou de la mousse d'un bouillon de fermentation, comprenant :
la fermentation d'une charge d'alimentation avec un microorganisme fermentant dans un bouillon de fermentation pour fabriquer un produit de fermentation, où le bouillon de fermentation comprend une transglycosidase
et où le produit de fermentation a un rendement plus élevé qu'une fermentation témoin manquant de la transglycosidase ;
dans lequel la transglycosidase a au moins 80% d'identité de séquence d'acides aminés avec l'enzyme transglucosidase mature de la SEQ ID NO : 1 et dans lequel la transglycosidase est produite par une expression dans un *Trichoderma.*

2. Procédé selon la revendication 1, comprenant en outre la récupération du produit de fermentation.

3. Procédé selon l'une quelconque des revendications précédentes, dans lequel le produit de fermentation est la lysine, l'acide lactique ou l'acide glutamique.

4. Procédé selon l'une quelconque des revendications précédentes, dans lequel l'organisme fermentant est *Brevibacterium flavum, Corynebacterium glutamicum* 10178, *Corynebacterium glutamicum* 10238 ou *Lactobacillus rhamnosus.*

5. Procédé selon l'une quelconque des revendications précédentes, dans lequel la transglycosidase comprend la SEQ ID NO : 1 ou a au moins 85%, 90%, 95%, 98% ou 99% d'identité de séquence d'acides aminés avec l'enzyme transglucosidase mature de la SEQ ID NO : 1.

6. Procédé selon l'une quelconque des revendications précédentes, dans lequel la charge d'alimentation comprend un produit de liquéfaction d'amidon, de l'amidon perlé, du glucose pur, du saccharose pur ou un sirop de sucre.

7. Procédé selon l'une quelconque des revendications précédentes, dans lequel la transglycosidase est une transglucosidase de *Trichoderma* (TrTG) et elle est présente à 1-6 kg/10³ kg (kg/t), 2-5 kg/10³ kg (kg/t) ou 2-4 kg/10³ kg (kg/t).

8. Procédé selon l'une quelconque des revendications précédentes, dans lequel :
(a) le produit de fermentation est la lysine et l'organisme fermentant est *Brevibacterium flavum* ; ou
(b) le produit de fermentation est la lysine, l'organisme fermentant est *Brevibacterium flavum* et la transglucosidase est une transglucosidase de A. *Niger* (TG) ; ou
(c) le produit de fermentation est l'acide glutamique et l'organisme fermentant est *Corynebacterium glutamicum* SP 10238 ; ou
(d) le produit de fermentation est l'acide lactique et l'organisme fermentant est *Lactobacillus rhamnosus.*

9. Procédé selon la revendication 8, dans lequel la charge d'alimentation comprend du glucose pur ou un sirop de sucre.

10. Utilisation d'une transglycosidase dans une fermentation pour réduire la viscosité d'un bouillon de fermentation par un microorganisme fermentant, dans laquelle la transglycosidase a au moins 80% d'identité de séquence d'acides aminés avec l'enzyme transglucosidase mature de la SEQ ID NO : 1.

11. Utilisation d'une transglycosidase dans une fermentation pour réduire la formation de mousse d'un bouillon de fermentation par un microorganisme fermentant, dans laquelle la transglycosidase a au moins 80% d'identité de séquence d'acides aminés avec l'enzyme transglucosidase mature de la SEQ ID NO : 1.

12. Utilisation selon la revendication 10 ou la revendication 11, dans laquelle la transglycosidase augmente le rendement d'un produit de fermentation par un microorganisme fermentant.

13. Utilisation selon l'une quelconque des revendications 10 à 12, dans laquelle la transglycosidase a au moins 98% d'identité de séquence d'acides aminés avec l'enzyme transglucosidase mature de la SEQ ID NO : 1.

14. Utilisation selon les revendications 10 à 13, dans laquelle la fermentation fabrique un produit de fermentation choisi dans le groupe constitué de lysine, acide citrique ou acide glutamique.

15. Utilisation selon l'une quelconque des revendications 10 à 14, dans laquelle l'organisme fermentant est choisi dans le groupe constitué de *Corynebacterium glutamicum* SP 10238, *Lactobacillus rhamnosus, Brevibacterium flavum* ou *Corynebacterium glutamicum* 10178.
